# EUROPEAN PATENT SPECIFICATION

(11) **EP 1 863 414 B1**
(45) Date of publication and mention of the grant of the patent: **28.03.2012**
(21) Application number: 06720960.1
(22) Date of filing: 23.02.2006
(51) Int. Cl.: A61F 2/42

(54) **PROSTHESIS FOR CORRECTION OF FLATFOOT DEFORMITY**
PROTHESE ZUR KORREKTUR VON PLATTFÜSSEN
PROTHÈSE POUR CORRIGER UNE MALFORMATION DE PIED PLAT

(30) Priority: 23.02.2005 US 655712 P
(43) Date of publication of application: 12.12.2007
(73) Proprietor: Parks, Brent, West Friendship, MD 21794-9520 (US); SHON, Lew, Baltimore MD 21218 (US); Chiodo, Christopher P., Walpole, MD 02081 (US)
(72) Inventor: Parks, Brent, West Friendship, MD 21794-9520 (US); SHON, Lew, Baltimore MD 21218 (US); Chiodo, Christopher P., Walpole, MD 02081 (US)
(74) Representative: Strandin, Heléne
(86) International application number: PCT/US2006/006217
(87) International publication number: WO 2006/091642

(56) References cited:
- WO-A-2004/032806
- FR-A- 2 645 735
- FR-A- 2 728 783
- US-A1- 2002 072 803

## Description

### BACKGROUND OF THE INVENTION

Flatfoot deformity, also known as pes planus, is a result of a loss of the normal medial longitudinal arch. As a result, the calcaneus bone may lie in valgus and external rotation relative to the talus. This deformity occurs in both children and adults and, in some cases, may limit normal function. Flatfoot deformity may cause pain at the foot or ankle, pain while walking or standing, or lower back and knee pain.

Many times, the symptoms of flatfoot deformity may be treated using non-surgical solutions such as orthotics, anti-inflammatory medications or ice and rest. However, sometimes the symptoms are too severe or remain even with these more conservative treatments. One option for surgical treatment of flatfoot deformity is the insertion of a prosthesis into to sinus tarsi opening. The prosthesis restores the correct arch of the foot by preventing the displacement of the talus bone and preventing pronation of the foot. Typically, this implant has been in the form of a conical or rounded shape internal prosthesis.

Despite the extensive development of internal human prostheses, they continue to exhibit certain disadvantages. For example, they are difficult to insert and remove by virtue of the shape and design of their base (which typically lack features for ease of removal, although the base is the end positioned closest to the surface after insertion). Thus, there exists a continuing need for the development of new and improved prostheses for the treatment of foot deformities such as flatfoot.

### SUMMARY OF THE INVENTION

Recognizing the need for the development of an improved implantable prosthesis for the correction of a human flatfoot deformity, the present invention is generally directed to alleviate the disadvantages of prior art devices as well as to provide other advantages over the prior art.

The present invention provides an internal human prosthesis for use in surgical procedures to treat flatfoot deformities, including pediatric, adult congenital, and adult acquired deformities. The internal prosthesis, designed for correction of a flatfoot deformity, is inserted between the human talus and calcaneus bones. The prosthesis preferably has a truncated conical shape along its long axis with a convex or tapered tip and a central bore through the center of its long axis. Advantageously, the base of the prosthesis includes a concave depression with a recess in its center.

When the prosthesis is inserted between the talus and calcaneus bones, the alignment of these bones is improved by virtue of the shape of the prosthesis. More particularly, the neck of the talus bone is elevated and the calcaneus is inverted. Once fully inserted, the prosthesis also prevents excessive eversion of the calcaneus bone.

### BRIEF DESCRIPTION OF THE DRAWINGS

The foregoing and other advantages and features of the invention will be more readily understood from the following detailed description of the invention provided below with reference to the accompanying drawings, in which:

FIG. 1A illustrates a bottom perspective view of a first embodiment of the present invention having a truncated pyramidal polyhedral shape;

FIG. 1B illustrates a side view of the first embodiment of the present invention;

FIG. 1C illustrates a bottom plan view of the first embodiment of the present invention;

FIG. 1D illustrates a cross-sectional view along line A of FIG. 1C;

FIG. 2A illustrates a bottom perspective view of the first embodiment of the present invention;

FIG. 2B illustrates a bottom perspective view of a second embodiment of the present invention, having a four-sided truncated pyramidal polyhedral shape;

FIGS. 2C-2E illustrate various views of a preferred embodiment of the present invention in the shape of a truncated cone;

FIG. 3A illustrates one set of typical dimensions for the prosthesis of the present invention; and

FIG. 3B illustrates a second set of typical dimensions for the prosthesis of the present invention.

### DETAILED DESCRIPTION OF THE INVENTION

In the following detailed description, reference is made to the accompanying drawings, which form a part hereof and show by way of illustration specific embodiments in which the invention may be practiced. These embodiments are described in sufficient detail to enable those skilled in the art to practice the invention, and it is to be understood that other embodiments may be utilized, and that changes may be made without departing from the scope of the present invention.

The present invention provides an internal prosthesis for correcting a flatfoot deformity that is simple to construct and use with low manufacturing costs, is more stable than other prostheses by virtue of its shape, and facilitates insertion, positioning, and removal by virtue of the unique shape of the concave base of the prosthesis with a polyhedral recess at its center.

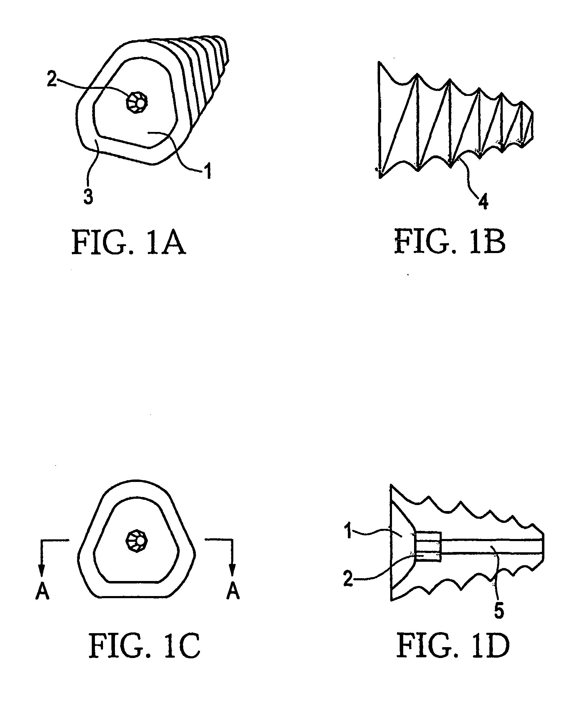

FIGS. 1A-1D illustrate a first embodiment of the prosthesis 10 in a bottom perspective view, a side view, a bottom plan view, and a cross-sectional view, respectively. The prosthesis 10 has a truncated pyramidal polyhedral shape along its long axis 8 with a base 6 that is comprised of or has within it a concave depression 1 with a polyhedral recess 2 in its center. The opposite end 7 of the prosthesis 10 is truncated and convex or tapered.

The surface of the concave depression 1 can either be smooth or include ridges to help guide a guide wire or drive toward the cannulation 5. The depth of the concave depression 1 may vary as well. The concave depression 1 may comprise the entire base 6 or may cover only a portion of the base 6. When the concave depression 1 comprises the entire base 6, lip 3 is not present. When the concave depression 1 comprises only a portion of the base 6, then lip 3 is present around the concave depression 1. The lip width may vary depending on the design considerations like the materials used for fabrication.

The base 6 of the prosthesis 10 preferably includes a polyhedral recess 2 in its center. This recess 2 is configured to receive a driving instrument which is used during insertion and/or removal of the prosthesis 10. The recess 2 may have a different number of sides, and be a shape such as a square, hexagonal, octagonal or a Trox drive design. In the preferred embodiment, the recess 2 is shaped to fit any standard driver, such as a 3.5mm hex driver. Additionally, the recess 2 may be tapered along the long axis 8 of the prosthesis 10 and the depth and diameter of the recess 2 may vary. One skilled in the art would appreciate that any shape, depth and diameter may be used for recess 2 as long as the recess 2 corresponds with the driver being used for insertion and/or removal of the prosthesis 10. Cannulation or central bore 5 is included along the long axis 8 of the prosthesis 10 in order to facilitate placement over a guide wire during insertion of the prosthesis.

The prosthesis 10 preferably includes threads 4 along its body. The threads 4 are configured for ease of insertion and retention of the prosthesis 10. The thread pitch and depth are matters of design choice, but will be such that the device is easily inserted and provides a predetermined resistance to being forced or "popped" back out of the intended location of the sinus tarsi during normal use. The thread pitch may be uniform or variable. Additionally, the threads 4 can be either continuous or interrupted (i.e., not one single continuous thread). Optimal designs will provide improved purchase with the sinus tarsi and better interdigitation of soft tissues. Alternatively, the threads may be replaced by ridges, protrusions, or slots/perforations.

In operation, the prosthesis 10 may be inserted over a guide wire or rod that is first placed in the region of the sinus tarsi, between the talus and calcaneus bones. The guide wire or rod is inserted between the neck of the talus bone and the anterior body of the calcaneus bone from lateral to medial using fluoroscopic guidance if necessary. The central bore 5 of the prosthesis 10 is then inserted over the wire using an inserter/removal device comprised of a handle, shaft, and polygonal head. The inserter/removal device may be any convenient wrench-type device. The polygonal head of the inserting device fits into a corresponding and similarly shaped recess 2 located in the center of the concave depression 1 on the base 6 of the prosthesis 10.

If the prosthesis 10 needs to be removed, removal is facilitated by virtue of the unique shape of the base 6 of the prosthesis with the polyhedral recess 2 at its center. More particularly, the head of the inserter/removal device can be mated with the polyhedral recess 2 more readily by virtue of the concave nature of the depression 1 within or comprising the base 6 of the prosthesis 10.

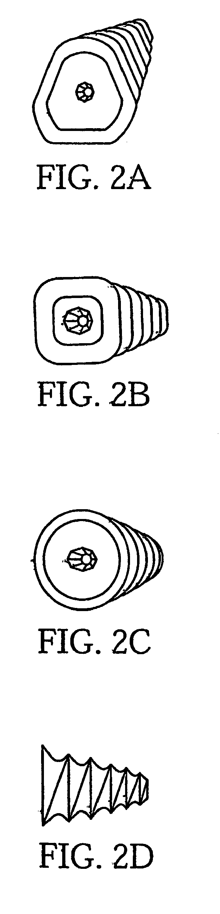

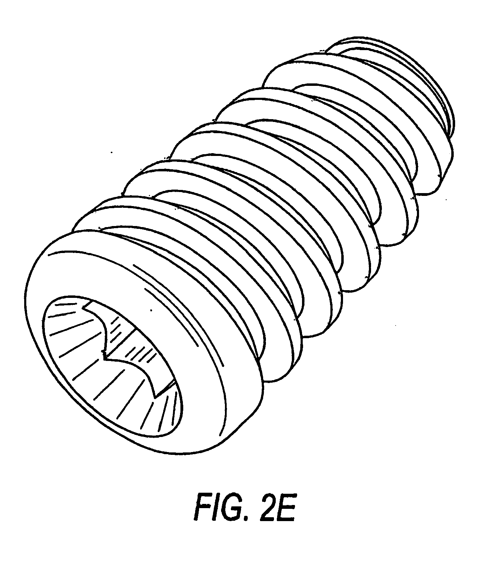

In the preferred embodiment, the shape of the prosthesis 10 is a truncated cone, as shown in FIGS. 2C, 2D and 2E. The prosthesis may also have a pyramidal polyhedral shape, as shown in FIGS. 2A and 2B, which allows better fill of the cavity in which the prosthesis 10 sits, and resists backing-out of the prosthesis 10. Tapered and pyramidal shapes require fewer sizes to accommodate anthropometric variable among patients.

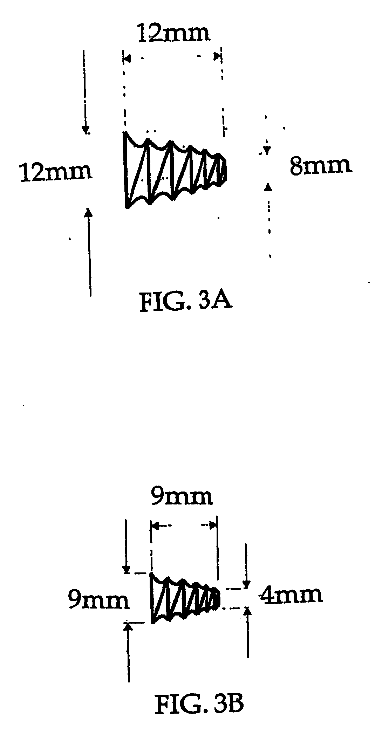

The size of the prosthesis 10 may vary to allow for accommodation of different patients. FIGS. 3A and 3B illustrate prostheses of two different sizes that would cover the majority of cases. The larger size in FIG. 3A would be appropriate for adults. The smaller size in FIG. 3B would be appropriate for children or small adults. One skilled in the are will readily appreciate how to select an appropriately shaped and sized prosthesis for a particular patient. Additionally, he/she would be able to readily determine how deep and at what orientation to insert the prosthesis, based on the alignment desired between the talus and calcaneus bones after insertion.

The prosthesis 10 may be composed of a metal, ceramic, polymer, bioresorbable, or biological material, including autograft, allograft, xenograft, or engineered tissue material. The material may also be modified with regard to radio-opacity in order to facilitate insertion, removal, and positioning.

Of course, one skilled in the art will appreciate how a variety of alternatives are possible for the individual elements, and their arrangement, described above, while still falling within the spirit of the invention. While the above describes several embodiments of the invention used primarily in connection with treating flatfoot disorder, those skilled in the art will appreciate that there are a number of alternatives, based on system design choices and choice of protocol options, and extensions that still fall within the spirit of the invention. Thus, it is to be understood that the invention is not limited to the embodiments described above, and that in light of the present disclosure, various other embodiments and applications should be apparent to persons skilled in the art. Accordingly, it is intended that the invention not be limited to the specific illustrative embodiments.

## Claims

1. A prosthesis for correcting a human flatfoot deformity comprising:
a body configured for insertion between the neck region of the talus bone and the calcaneus bone in a region of the sinus tarsi, wherein the body is oriented such that, when inserted, a long axis of the body approximately parallels a medial-lateral human anatomical axis, and wherein the body has a base end provided with a concave depression and an end opposite the base, and wherein the body is tapered along its long axis such that a cross-sectional area of the end of the body opposite the base is smaller than a cross-sectional area of the body at the base.

2. The prosthesis of claim 1, wherein the body is formed in the shape of a truncated cone.

3. The prosthesis of claim 1, wherein the body is formed in the shape of a truncated pyramidal polyhedral.

4. The prosthesis of claim 1, wherein the depression has a cross-sectional area which is smaller or equal to a cross-sectional area of the base.

5. The prosthesis of claim 4, wherein the concave depression has a variable depth along the long axis of the body.

6. The prosthesis of claim 5, wherein the concave depression may comprise at least one groove or ridge, thereby facilitating insertion and/or removal of the prosthesis.

7. The prosthesis of claim 6, wherein the concave depression has a uniform or tapered polyhedral recess, the recess being contiguous with a center of the concave depression and the center of the recess being oriented along the long axis of the body, further facilitating the insertion and/or removal of the prosthesis.

8. The prosthesis of claim 1, wherein the end opposite the base is truncated.

9. The prosthesis of claim 1, wherein the end opposite the base is convex or tapered.

10. The prosthesis of claim 1, further comprising an axial bore oriented along the long axis of the body.

11. The prosthesis of claim 1, further comprising threads along at least one portion of a surface of the prosthesis between the base and the end opposite the base, thereby facilitating soft-tissue apposition, interdigitation, and adherence.

12. The prosthesis of claim 11, wherein the threads are continuous.

13. The prosthesis of claim 11, wherein the threads are interrupted.

14. The prosthesis of claim 11, wherein the threads have variable pitch.

15. The prosthesis of claim 11, wherein the threads have uniform pitch.

16. The prosthesis of claim 11, wherein the threads have variable width and height.

17. The prosthesis of claim 11, wherein the threads have uniform width and height.

18. The prosthesis of claim 11, wherein the threads have one of a group of variable or uniform shapes including substantially v-shaped, right triangle, and sinusoidal.

19. The prosthesis of claim 1, comprising at least one of metal, ceramic, polymer, bioresorbable, or biological material, including autograft, allograft, xenograft, or engineered tissue material.

20. The prosthesis of claim 19, comprising a material that may be modified with regard to radio-opacity, thereby facilitating insertion, removal, and positioning.

## Patentansprüche

1. Prothese zur Korrektur von Plattfüßen beim Menschen, umfassend:
einen Körper, der für das Einsetzen zwischen dem Halsbereich des Sprungbeins und dem Fersenbein in einem Bereich des Sinus tarsi eingerichtet ist, wobei der Körper so ausgerichtet ist, dass, wenn er eingesetzt ist, eine Längsachse des Körpers ungefähr parallel zu einer mediolateralen anatomischen Achse des Menschen verläuft, und wobei der Körper ein Basisende aufweist, das mit einer konkaven Senkung versehen ist, und ein Ende gegenüber der Basis, und wobei der Körper entlang seiner Längsachse derart verjüngt ist, dass eine Querschnittsfläche des Endes des Körpers gegenüber der Basis kleiner ist als eine Querschnittsfläche des Körpers an der Basis.

2. Prothese nach Anspruch 1, wobei der Körper in Form eines Kegelstumpfs geformt ist.

3. Prothese nach Anspruch 1, wobei der Körper in Form eines abgestumpften pyramidenförmigen Polyeders geformt ist.

4. Prothese nach Anspruch 1, wobei die Senkung eine Querschnittsfläche aufweist, die kleiner als eine oder gleich einer Querschnittsfläche der Basis ist.

5. Prothese nach Anspruch 4, wobei die konkave Senkung entlang der Längsachse des Körpers eine veränderliche Tiefe aufweist.

6. Prothese nach Anspruch 5, wobei die konkave Senkung mindestens eine Nut oder Rille aufweisen kann, wodurch das Einsetzen und/oder Herausnehmen der Prothese vereinfacht wird bzw. werden.

7. Prothese nach Anspruch 6, wobei die konkave Senkung eine gleichmäßige oder verjüngte polyedrische Vertiefung aufweist, wobei die Vertiefung an einen Mittelpunkt der konkaven Senkung angrenzt und der Mittelpunkt der Vertiefung entlang der Längsachse des Körpers ausgerichtet ist, wodurch das Einsetzen und/oder Herausnehmen der Prothese weiter vereinfacht wird bzw. werden.

8. Prothese nach Anspruch 1, wobei das Ende gegenüber der Basis abgestumpft ist.

9. Prothese nach Anspruch 1, wobei das Ende gegenüber der Basis konvex oder verjüngt ist.

10. Prothese nach Anspruch 1, ferner umfassend eine axiale Bohrung, die entlang der Längsachse des Körpers ausgerichtet ist.

11. Prothese nach Anspruch 1, ferner umfassend Gewindegänge entlang mindestens einem Abschnitt einer Fläche der Prothese zwischen der Basis und dem Ende gegenüber der Basis, wodurch die Anlagerung, Verzahnung und Anhaftung von Weichgewebe vereinfacht werden.

12. Prothese nach Anspruch 11, wobei die Gewindegänge durchgehend sind.

13. Prothese nach Anspruch 11, wobei die Gewindegänge unterbrochen sind.

14. Prothese nach Anspruch 11, wobei die Gewindegänge eine unterschiedliche Steigung aufweisen.

15. Prothese nach Anspruch 11, wobei die Gewindegänge eine einheitliche Steigung aufweisen.

16. Prothese nach Anspruch 11, wobei die Gewindegänge eine veränderliche Breite und Höhe aufweisen.

17. Prothese nach Anspruch 11, wobei die Gewindegänge eine einheitliche Breite und Höhe aufweisen.

18. Prothese nach Anspruch 11, wobei die Gewindegänge eine Form aus einer Gruppe veränderlicher oder gleichmäßiger Formen einschließlich im Wesentlichen V-förmig, der Form eines rechtwinkligen Dreiecks und sinusförmig aufweisen.

19. Prothese nach Anspruch 1, umfassend ein metallisches, keramisches, polymeres, bioresorbierbares und/oder biologisches Material einschließlich körpereigenen, artgleichen, artfremden oder künstlich hergestellten Gewebematerials.

20. Prothese nach Anspruch 19, umfassend ein Material, das hinsichtlich der Röntgensichtbarkeit verändert werden kann, wodurch das Einsetzen, Herausnehmen und Platzieren vereinfacht werden.

## Revendications

1. Prothèse pour corriger une malformation de pied plat d'un être humain comprenant :
un corps configuré pour être inséré entre la région du col du talus et le calcanéus dans une région du sinus du tarse, dans laquelle le corps est orienté de telle sorte que, quand il est inséré, un axe longitudinal du corps est approximativement parallèle à un axe anatomique humain médian-latéral, et dans laquelle le corps présente une extrémité de base dotée d'un enfoncement concave et une extrémité opposée à la base, et dans laquelle le corps est effilé le long de son axe longitudinal de sorte qu'une aire de la section de l'extrémité du corps opposée à la base est plus petite qu'une aire de la section du corps au niveau de la base.

2. Prothèse selon la revendication 1, dans laquelle le corps est formé sous forme d'un cône tronqué.

3. Prothèse selon la revendication 1, dans laquelle le corps est formé sous forme d'un polyèdre pyramidal tronqué.

4. Prothèse selon la revendication 1, dans laquelle l'enfoncement présente une aire de la section qui est inférieure ou égale à une aire de la section de la base.

5. Prothèse selon la revendication 4, dans laquelle l'enfoncement concave présente une profondeur variable le long de l'axe longitudinal du corps.

6. Prothèse selon la revendication 5, dans laquelle l'enfoncement concave peut comprendre au moins une rainure ou crête, facilitant ainsi l'insertion et/ou le retrait de la prothèse.

7. Prothèse selon la revendication 6, dans laquelle l'enfoncement concave présente un évidement polyédrique régulier ou conique, l'évidement étant contigu à un centre de l'enfoncement concave et le centre de l'évidement étant orienté le long de l'axe longitudinal du corps, facilitant encore plus l'insertion et/ou le retrait de la prothèse.

8. Prothèse selon la revendication 1, dans laquelle l'extrémité opposée à la base est tronquée.

9. Prothèse selon la revendication 1, dans laquelle l'extrémité opposée à la base est convexe ou effilée.

10. Prothèse selon la revendication 1, comprenant en outre un trou axial orienté le long de l'axe longitudinal du corps.

11. Prothèse selon la revendication 1, comprenant en outre des filets le long d'au moins une portion d'une surface de la prothèse entre la base et l'extrémité opposée à la base, facilitant ainsi l'apposition, l'interdigitation et l'adhérence des tissus mous.

12. Prothèse selon la revendication 11, dans laquelle les filets sont continus.

13. Prothèse selon la revendication 11, dans laquelle les filets sont interrompus.

14. Prothèse selon la revendication 11, dans laquelle les filets présentent un pas variable.

15. Prothèse selon la revendication 11, dans laquelle les filets présentent un pas régulier.

16. Prothèse selon la revendication 11, dans laquelle les filets présentent une largeur et une hauteur variables.

17. Prothèse selon la revendication 11, dans laquelle les filets présentent une largeur et une hauteur régulières.

18. Prothèse selon la revendication 11, dans laquelle les filets présentent une forme parmi un groupe de formes variables ou régulières comprenant essentiellement en forme de V, de triangle rectangle, et sinusoïdale.

19. Prothèse selon la revendication 1, comprenant au moins un matériau parmi les matériaux métalliques, céramiques, polymères, biorésorbables ou biologiques, comprenant les autogreffes, allogreffes, xénogreffes, ou matériaux issus de l'ingénierie tissulaire.

20. Prothèse selon la revendication 19, comprenant un matériau qui peut être modifié en ce qui concerne l'opacité aux radiations, facilitant ainsi l'insertion, le retrait et le positionnement.
